(19) Europäisches Patentamt
European Patent Office
Office européen des brevets

(11) EP 1 916 251 A1

(12) # EUROPEAN PATENT APPLICATION
published in accordance with Art. 153(4) EPC

(43) Date of publication:
**30.04.2008 Bulletin 2008/18**

(21) Application number: **06775252.7**

(22) Date of filing: **01.08.2006**

(51) Int Cl.:
***C07F 9/08*** *(2006.01)* ***C07C 43/235*** *(2006.01)*
***A61K 31/09*** *(2006.01)* ***A61P 35/00*** *(2006.01)*

(86) International application number:
**PCT/CN2006/001918**

(87) International publication number:
**WO 2007/014524 (08.02.2007 Gazette 2007/06)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.08.2005 CN 200510089004**

(71) Applicant: **Zhe Jiang Cell Biomedical Research Co., Ltd.**
**Tian Tai Town, Tai Zhou**
**Zhejiang 317 200 (CN)**

(72) Inventors:
- **LI, Yiping**
  **Taizhou, Zhejiang 317200 (CN)**
- **LI, Ning**
  **Taizhou, Zhejiang 317200 (CN)**

(74) Representative: **Le Coupanec, Pascale A.M.P.**
**Nony & Associés,**
**3 rue de Penthièvre**
**75008 Paris (FR)**

(54) **ERIANIN SALTS, THEIR PREPARATION METHODS AND PHARMACEUTICAL COMPOSITIONS CONTAINING THE SAME**

(57) This invention relates to a kind of Erianin salt and the preparing process thereof. The said Erianin salt is a compound with the following general formula (I), wherein R is the salt formed by monobasic acid radical of inorganic oxacid combining with metals, ammonium salts, organic amine. This invention also relates to a pharmaceutical composition comprising Erianin salt. Compared with Erianin, the said Erianin salt has far better solubility, which can improve the bioavailability and show better antineoplastic efficacy.

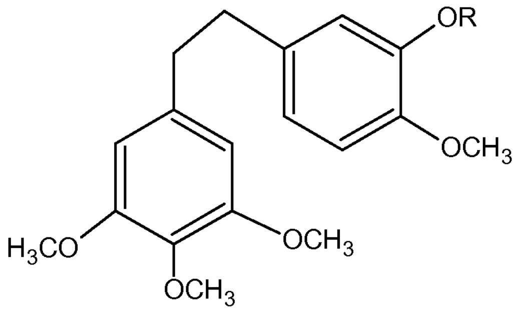

(I)

EP 1 916 251 A1

## Description

### FIELD OF THE INVENTION

The present invention relates to a salt of Erianin and the preparing method thereof.

The present invention also relates to a pharmaceutical composition comprising the compound.

### BACKGROUND OF THE INVENTION

Cancer is considered to be the worst disease except the cardiovascular disease. At present chemotherapy and radiotherapy are frequently used for treatment of cancer, but their toxicant and side effect is very adverse for health. The specialists home and abroad have found that dendrobium, a kind of traditional Chinese medicine, can be antineoplastic, anti-aging and expanding blood vessel, and its extract by ethanol and the bibenzyl compounds have antineoplastic activity *in vivo* of different degrees. The active component of dendrobium has caught the attention of the world.

The findings of Wang Tianshan (In vitro Inhibition Activities on the Growth of Tumor Cell Strain K256 by Constituents from Dendrobium Chrysotoxum, Natural Product Research and Development, 1997, 9 (2), 1~3*)* showed that bibenzyls and phenanthrenes have inhibitory effect on the assembling and caryocinesis of the *in vitro* cultured murine microtubulin $L_{1210}$, , $P_{388}$ cell strain and many human tumor cell strains including A-549, MCF-7, HT-29, SKMEL-5, MLM, SK-OV-3, and HL-60. The dihydrobibenzyls and phenanthrenes in Dendrobium chrysotoxum have different degrees of inhibitory effect on the growth of tumor cell strain $K_{256}$, among which Erianin (dihydrocombretastatin A-4) is the most active. The chemical structural formula of Erianin is as follows:

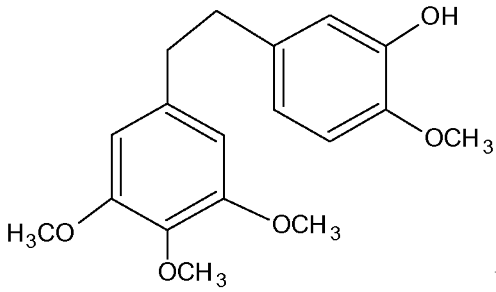

Erianin

Erianin has the best effect on the liver cancer of mouse, its tumor inhibitory rate being 50.82%. Related study *(*Inhibitory effects of dendrobium chrysotoxum and its constituents on the mouse HePA and ESC, Journal of China Pharmaceutical University, 1994, 25 (3), 188~189*)* infers that the side effect of Erianin is far lower then 5FU, the medicine for chemotherapeutics of cancer. Erianin have negative proton effect to many kinds of cancer cells, the effecting target being the microtubulin in the cell. It can inhibit the polymerizing of microtubulin, stimulate the hydrolization of microtubulin-dependent GTP, and competitively bind with protein with colchicine. The study of Li Yunman (Erianin Induces Apoptosis of Human leukemia HL-60 cells, Acta Pharmacologica Sinica, 2001,Nov,22(11),1018-1022*)* showed that Erianin significantly inhibited the growth of human leukemia HL-60 cells. The inhibition might be the result of the induced apoptosis and the altered expression of bcl-2 and bax genes in HL-60 cells.

As the water-solubility of Erianin is low, the solubility in water being just <1mg/ml, its preparation performance and clinical application are limited, so it is necessary to study the salts that can improve its water-solubility. At present, there is not any literature on the salts of Erianin and it's preparing process.

### SUMMARY OF THE INVENTION

One object of this invention is to provide a pharmaceutically acceptable salt of Erianin (hereinafter referred to as Erianin salt). The compound provided in this invention has preferable antineoplastic activity, and better water-solubility compared with Erianin, so it is convenient to get the preparation, and higher in vivo bioavailability can be achieved.

The other object of this invention is to provide a process for preparing the Erianin salt.

Another object of this invention is to provide a parmaceutical composition comprising the Erianin salt.

According to one aspect of this invention, a kind of Erianin salt is provided in this invention. The said Erianin salt is a compound of the general formula (I) as follows:

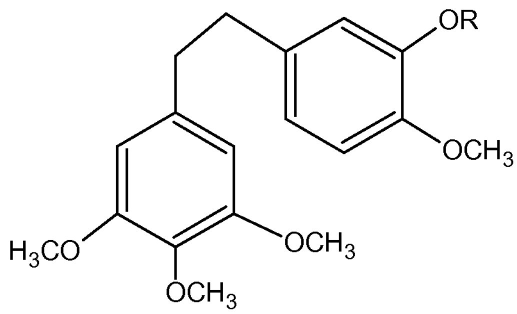

(I)

wherein:

R is the salt formed by monobasic acid radical of inorganic oxacid combining with metals, ammonium salts, or organic amine;
said metals are selected from a group consisting of alkali metals and alkali-earth metals;
said inorganic oxacid is selected from a group consisting of phosphoric acid, sulphuric acid, sulfurous acid, nitric acid, carbonic acid, hypochloric acid, trifluoroacetic acid, and sulfonic acid.

The general formula of said sulfonic acid is $R-SO_3H$, wherein R is hydrocarbyl. The said sulfonic acid includes but is not limited to benzenesulfonic acid, para-toluenesulfonic acid, 2-naphthol sulfonic acid, methylsulfonic acid, ethylsulfonic acid, and 2-hyroxyethylsulfonic acid.
The said alkali metals include sodium, potassium, etc; the said alkali-earth metals include magnesium, calcium, etc.
The said organic amine is selected from a group consisting of triethylamine, 3-(2-hydroxyl)-amine, N-ethylpiperidine, N,N-diethyl-piperazidine, etc.
The said Erianin salt is prepared by esterifying Erianin with the above-mentioned acids and then reacting with metals, ammonium salts or organic amine.
The Erianin salt can be a prodrug of Erianin to increase the water-solubility for better absorption and utilization in the human body.
The said inorganic oxacidoate salt of Erianin in this invention is prepared by esterifying Erianin with the above-mentioned inorganic oxacid and then reacting with metals, ammonium salts, or organic amine.
Further, the said Erianin salt is an organic phosphate salt of Erianin or a sulphate salt of Erianin.
Preferably, R is a phosphate radical, so the said Erianin salt is an organic phosphate salt of Erianin, whose general formula is shown in (II) below:

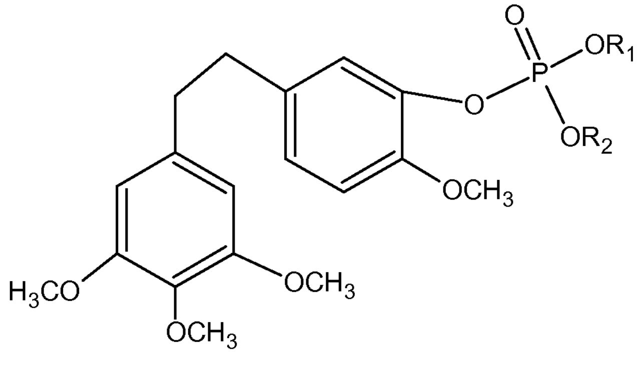

(II)

wherein $R_1$, $R_2$ are independently H, Na, K, or $NH_4$ respectively, and $R_1$ and $R_2$ are not H simultaneously.
More preferably, both $R_1$, $R_2$ are Na, and the said organic phosphate salt of Erianin is disodium phosphate of

Erianin.

Preferably, R can be sulfate, so the said Erianin salt is the sulphate salt of Erianin, whose general formula is shown in (III) below:

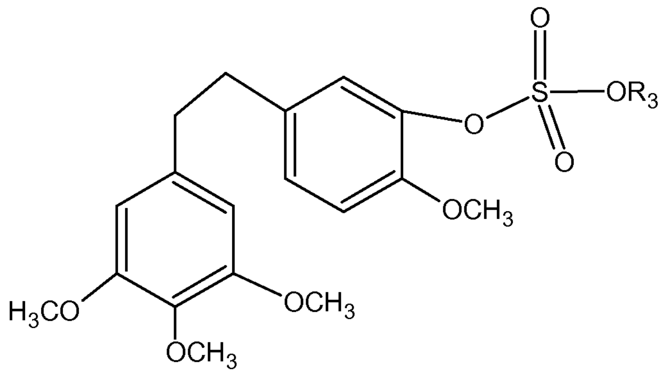

(III)

wherein, $R_3$ is Na, K, or $NH_4$.

Most preferably, $R_3$ is Na, and the sulphate salt of Erianin is sodium sulphate of Erianin.

According to the other aspect of this invention, a process of preparing this kind of compound is provided in this invention.

Those skilled in the art can use the common preparative method, with Erianin as a starting material, to prepare the above compound (I). The preparative process provided in this invention is described below:

The process for preparing the organic phosphate salt of Erianin comprises the following steps:

A1. reacting Erianin with phosphorylating agent by phosphorylating reaction of the phenolic hydroxyl in the presence of an acid binding agent to form compound (IV), an intermediate of the phosphorylated Erianin, the reacting temperature being 25~80°C and the reacting solvent being an inert organic solvent;
A2. reacting the reaction liquid obtained in step A1 with alkali directly to afford compound (II), an organic phosphate salt of Erianin;
the synthetic route is:

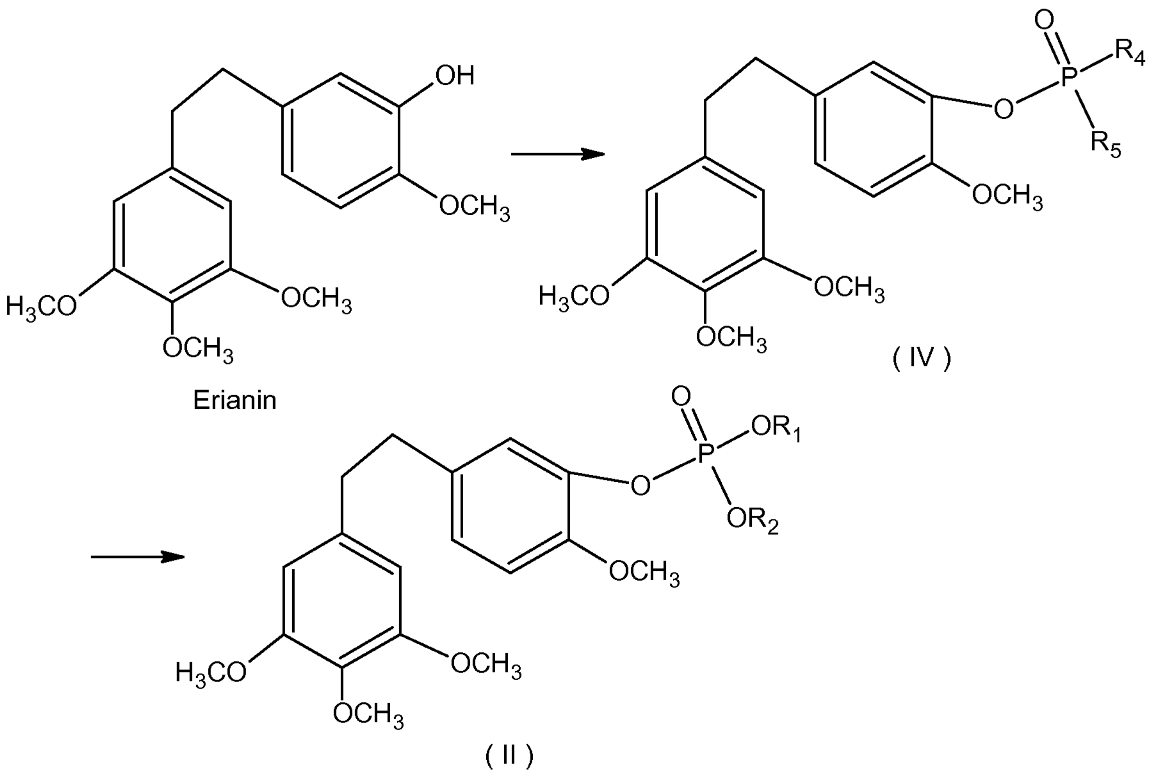

Erianin

( IV )

( II )

wherein
in formula (IV), $R_4$, $R_5$ are independently Obu$^t$, Cl, or Br respectively, wherein bu$^t$ represents tert-butyl group;
in formula (II), R1, R2 are independently H, Na, K, or $NH_4$ respectively, and $R_1$ and $R_2$ are not H simultaneously.

The two steps above can be reacted continuously, which means that the intermediate (IV) obtained in step A1 can be reacted with the reagent of the next step directly without further separation and purification.

Further, the two steps of the preparing process above are:

A1. reacting Erianin with excess phosphorylating agent by phosphorylating reaction of the phenolic hydroxyl in the presence of acid binding agent, stirring thoroughly for 2~12 hours, to form compound (IV), an intermediate of the phosphorylated Erianin was obtained, the reacting temperature being 25~80°C and the reacting solvent being an inert organic solvent;
A2. evaporating the solvent in the reaction liquid obtained in step A1 under reduced pressure, adding dropwise 1~6 mol/L of inorganic alkali to the reaction liquid while stirring, then stirring 2~24 hours at 0~90°C, removing the solvent under reduced pressure to obtain the crude product of compound (II), and recrystallizing the crude product with recrystallizing solvent to obtain the pure product of organic phosphate salt of Erianin (yield: 79~95%, melting point: 266~269 °C (decomposed));

In one embodiment, in step A1 :

said phosphorylating agent is one or more kinds of pentavalent phosphorous oxyhalides selected from a group consisting of $OPCl_3$, $OPBr_3$, and $(Bu^tO)_2P(O)Cl$.
said acid binding agent is one or more kinds of reagents selected from a group consisting of pyridinium, monalkylamine, dialkylamine, trialkylamine, etc.

Further, the acid binding agent is added dropwise into the reaction liquid when stirred thoroughly; while the reaction is completed, washing the reaction mixture by water, separating it, drying the organic layer by anhydrous sodium sulfate, and evaporating the solvent under reduced pressure.
In one embodiment, in step A1 :

said inert organic solvent is one or more kinds of solvents selected from a group consisting of dichloromethane, dichloroethane, benzene, carbon tetrachloride, acetonitrile, etc.

In one preferred embodiment of this invention, the reacting temperature in step A1 is room temperature.

In one embodiment, the process of the invention in step A2 comprises:

adding alkali dropwise into the reaction liquid with full stirring, preferably continuing to stir 8~10 hours at 30~80°C, then cooling the reaction liquid, removing the insoluble substance by filtering. Distilling most solvent under reduced pressure to get the solid crude product, which was recrystallized by using organic solvent to afford the objective product (II).

In one embodiment, in step A2, said alkali is selected from a group consisting of NaOH, KOH, $NaOCH_3$, $KOCH_3$, $NaOCH_2CH_3$, $KOCH_2CH_3$, etc. The concentration of alkali can be prepared to be 1~6 mol/L on the basis of the quantity and volume needed.

Said recrystallizing solvent of objective product (II) is selected from a group consisting of water, methanol, ethyl alcohol, dimethyl carbinol, and acetone, and the mixture of methanol and acetone is preferred.

In one preferred embodiment of this invention, with regard to the preparing process above mentioned, in said step A1, the phosphorylating agent is $OPCl_3$, and the acid binding agent is triethylamine; in said step A2, the alkali is NaOH.

When reacting Erianin with phosphorylating agent such as dibenzyl phosphorite ($(BnO)_2P(O)H$, bn represents benzyl group), the intermediate dibenzyl phosphate of Erianin can be formed. The intermediate can react first with NaI to form phosphate of Erianin, then with alkali to form salt, Sc.

In one embodiment, another preparing process of a pharmaceutically acceptable salt of Erianin and in particular a phosphate salt of Erianin is provided in this invention, comprising the follow steps:

B1. under the protection of inert gas, reacting Erianin with dibenzylphosphorite in the presence of acid binding agent by phosphorylating reaction of the phenolic hydroxyl group to obtain compound (V), the reaction temperature being -40°C~10°C, the reaction is carried out in inert organic solvent;

B2. under the protection of inert gas, reacting compound (V) with NaI in the presence of active group protection agent to obtain compound (VI), the reaction is carried out in inert organic solvent;

B3. reacting compound (VI) with alkali to obtain compound (II),

the synthetic route is:

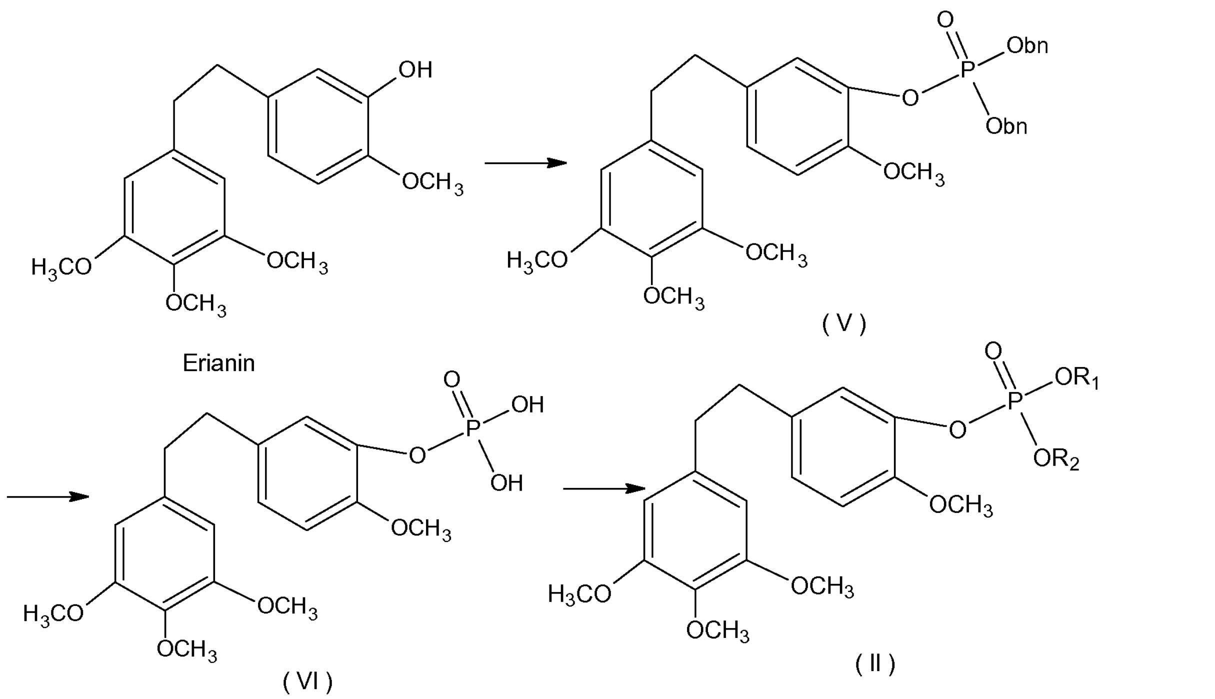

Erianin

( V )

( VI )

( II )

wherein in formula (V), bn represents benzyl group; in formula (II), $R_1$, $R_2$ are independently H, Na, K, or $NH_4$ respectively, and $R_1$ and $R_2$ are not H simultaneously.

In one embodiment, in step B1:

said dibenzyl phosphate is phosphorylating agent.
in one embodiment, in step B1:

said acid binding agent is selected from a group consisting of pyridinium, monalkylamine, dialkylamine, trialkylamine, etc. In one embodiment of this invention, the acid binding agent is diisopropylethylamine and dimethylamino pyridine (DMAP).

In one embodiment, in step B1:

said inert gas is argon, nitrogen gas or carbon monoxide, etc. In one embodiment of this invention, the inert gas is argon.

In one embodiment, in step B1:

said inert organic solvent is selected from a group consisting of dichloromethane, dichloroethane, benzene, carbon tetrachloride, acetonitrile, etc. In one embodiment of this invention, the inert organic solvent is acetonitrile and carbon tetrachloride.

In one embodiment, in step B2:

said inert gas is argon, nitrogen gas or carbon monoxide, etc. In one embodiment of this invention, the inert gas is argon.

In one embodiment, in step B2:

said inert organic solvent is selected from a group consisting of dichloromethane, dichloroethane, benzene, carbon tetrachloride, acetonitrile, etc. In one embodiment of this invention, the said inert organic solvent is acetonitrile and/or carbon tetrachloride;

In one embodiment, in step B2, the active group protection agent is trimethyl chloride silane, etc.
In one embodiment, in step B3:

said alkali is selected from a group consisting of NaOH, KOH, $NaOCH_3$, $KOCH_3$, $NaOCH_2CH_3$ and $KOCH_2CH_3$. In one embodiment of this invention, the alkali is sodium hydroxide.

In one preferred embodiment of this invention, when synthesizing the intermediate (V) with Erianin, dissolving Erianin in acetonitrile under argon protection, adding $CCl_4$ at -25°C and stirring for 5 minutes, adding dropwise diisopropylethylamine and DMAP with syringe, adding dibenzyl phosphorite 1 minute later, and keeping the reacting temperature under -10°C; when forming the phosphate of Erianin from intermediate (V), under the protection of argon, dissolving intermediate (V) and NaI with acetonitrile, stirring vigorously, adding trimethyl chlorosilane slowly, stirring for 20 minutes, and the phosphate of Erianin (V) is obtained. Reacting the phosphate of Erianin (VI) with sodium methoxide to get a colorless crystal (II).
In one embodiment, the invention provides another process for preparing a pharmaceutically acceptable salt of Erianin, in particular a sulphate salt of Erianin, comprising the following steps:

C1. reacting Erianin with halo sulfonic acid by sulfonylating reaction of phenolic hydroxyl in the presence of an acid binding agent to obtain compound (VII), an intermediate of the sulfonylated Erianin, the reacting temperature being -5~-10°C and the reacting solvent being an inert organic solvent;
C2. reacting the reacting liquid obtained in step C1 with alkali directly to prepare compound (III), a sodium sulphate of Erianin;

The synthetic route is:

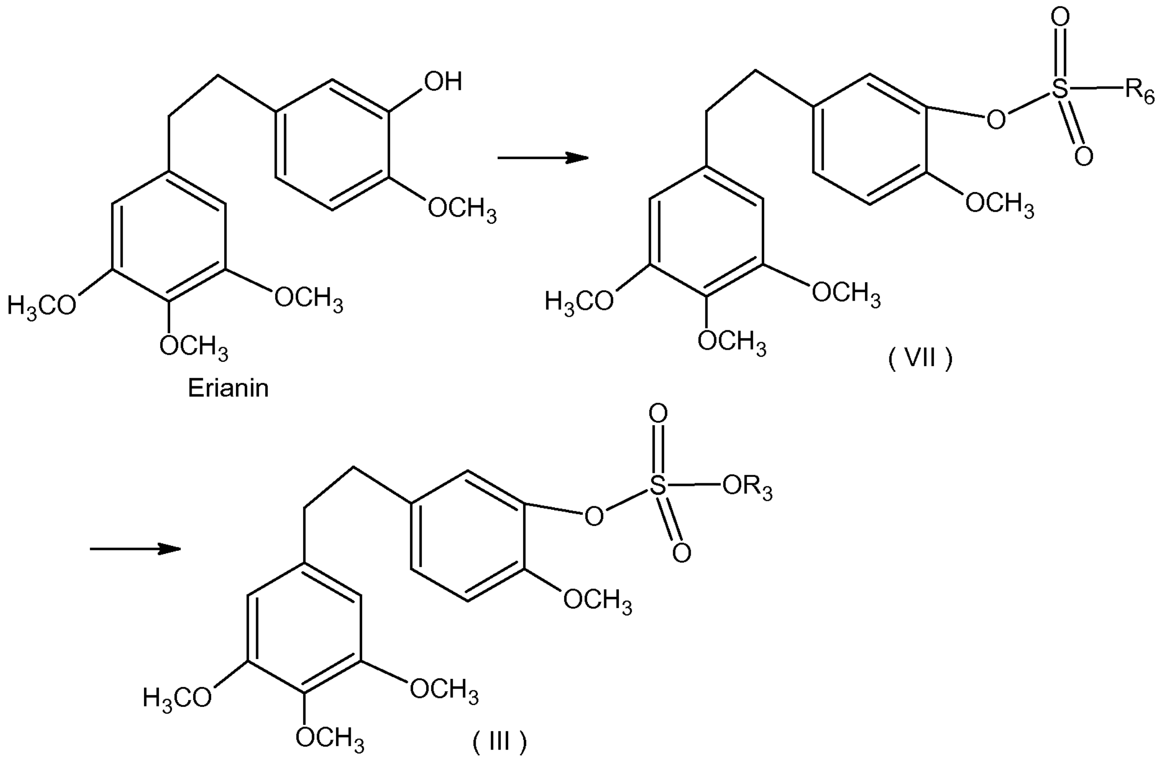

Erianin

( VII )

( III )

wherein in formula (VII), $R_6$ is Cl or Br, etc; in formula (III) $R_3$ is Na, K, or $NH_4$.

Specifically, the above-mentioned preparing process comprises two following steps:

C1. reacting Erianin with halo sulfonic acid by sulfonylating reaction of phenolic hydroxyl in the presence of acid binding agent, stirring fully for 1 to 10 hours to obtain compound (VII), an intermediate of sulfonylated Erianin, the reacting temperature being -5~-10°C and the reacting solvent being an inert organic solvent;

C2. adjusting the pH of the reaction liquid obtained in step C1 to 8~12 with alkali of the concentration of 5-15 mol/L while thoroughly stirring, filtering after cooling, washing with dry ether and drying, dissolving the solid product in methanol, passing through column, and evaporating the solvent to get sodium sulphate of Erianin, the reacting temperature being room temperature.

In one embodiment, in step C1:

said halogen sulfonic acid is chlorosulfonic acid or bromo sulfonic acid;

said acid binding agent is selected from a group consisting of pyridinium, monalkylamine, dialkylamine, tri alkylamine, etc;

said inert organic solvent is selected from a group consisting of dichloromethane, dichloroethane, benzene, carbon tetrachloride, etc.

Further, in step C1, adding Erianin, N,N-dimethylaniline in a round bottom flask, adding dichloromethane of the same volume of N,N-dimethylaniline, stirring in ice-salt bath(-5~-10°C), adding dropwise chlorosulfonic acid, and continuing to stir for 1 hour in brine bath.

In one embodiment, in step C2:

the alkali is selected from a group consisting of NaOH, KOH, $NaOCH_3$, $KOCH_3$, $NaOCH_2CH_3$, and $KOCH_2CH_3$, etc. The concentration of alkaline can be adjusted on the basis of the quantity and volume needed.

Further, in step C2: removing the brine bath, stirring for 24 hours at room temperature, adjusting the pH value of the reaction liquid to 10 with NaOH solution (10 mol/L) while stirring, cooling and filtering, washing with anhydrous ether, dissolving the solid product in methanol and purified by column chromatography after drying, and evaporating the solvent to get sodium sulfate of Erianin.

In one preferred embodiment of this invention, with regard to the above preparing process, in step C1: the said halo sulfonic acid is chlorosulfonic acid, the said acid binding agent is N, N-dimethylaniline, and the inert organic solvent is dichloromethane; in step C2, the alkali is sodium hydroxide.

It is another object of this invention is to provide a pharmaceutical composition comprising an efficient amount of Erianin salt as an active ingredient in amount effective to treat diseases together with pharmaceutically acceptable carrier and/or excipient.

Further, the said pharmaceutical composition is in the dosage form for injection or oral administration.

By using Erianin salt as the effective component, adding pharmaceutically acceptable accessories, and using the common method of this field, a pharmaceutical preparation of this invention can be made. The dosage forms of the composition can be: oral administration form, such as tablet, capsule (including hard capsule, soft capsule, enteric-coated capsule and microcapsule), powder, granules and syrups; non-oral administration form, such as injection, suppositories, pill, gelata and patch. Besides these common forms, the orally taken speed-release preparation (such as tablet, granules) and the orally or non-orally taken sustained-release preparation (such as tablet, granules, refined granules, pill, capsule, syrup, stable suspension, suspension, solution) can also be used in current invention, and preparations thereof are available by common methods. The preparation in current invention can be coated or non-coated according to the need. The injection form of Erianin salt is preferred in this invention.

The pharmaceutical accessories in this invention include those for solid preparation, such as excipient, lubricant, cohesives, disintegrant, stabilizer, blowing agent, coating agent, etc; or those for semisolid preparation and liquid preparation, such as solvent, solubilizing agent, suspending agent, isotonizing agent, buffer, emollient, emulsifier, etc. In addition, other pharmaceutical additives, such as preservative, antioxidant, colorant, edulcorant, correctant, etc. can also be used if necessary.

The dosage of pharmaceutical preparation can be chosen according to the subject of treating, dosage form of medicine, and means of administration.

Compared with Erianin, the Erianin salt of the present invention has much better water-solubility, so the preparation is easier and the *in vivo* bioavailability is higher, which means that there will be better antineoplastic effect. In current invention, "cancer" refers to all malignant tumors.

Experiment of pharmacodynamics indicated that disodium phosphate Erianin has conspicuous antineoplastic effect, the efficacy being the same either administered daily or every other day. This means that disodium phosphate Erianin is an anticancer compound of high efficacy and low toxicity. Disodium phosphate Erianin used together with other chemotherapeutics can apparently raise the lethality of chemotherapeutics on tumors.

An object of the invention is to provide a use of the pharmaceutically acceptable salt of Erianin of the invention in preparing an antitumor pharmaceutical.

The result of safety evaluation showed that the LD50 of disodium phosphate of Erianin is 1243.0377 mg/kg, indicating that disodium phosphate Erianin is very safe in clinical medication.

The advantages of the Erianin salt and its preparing process provided in this invention are as follows:

1. The Erianin salt provided in the present invention has better water-solubility than Erianin, so its preparation is easier. For instance, high concentration preparation for injection can be prepared, and the in vivo bioavailability of the medication can be improved.
2. The Erianin salt provided in present invention has an anticancer effect of high efficacy and low toxicity.
3. In the preparing process of the present invention, all reagents are ordinary products of chemical industry that are cheap and easy to obtain, and the route of synthesis is short and the operation is easy, suitable for industrial mass production.

In order to comprehend the invention better, the following description of preferred embodiments is to describe but not to limit the invention.

BRIEF DESCRIPTION OF THE DRAWINGS

FIG. 1 is the nuclear magnetic resonance analysis spectra ($^1$H NMR) of Erianin phosphate prepared in the present invention;

FIG. 2 is the nuclear magnetic resonance analysis spetra ($^{13}$C NMR) of Erianin phosphate prepared in the present invention;

FIG. 3 is the infrared analysis spectra of Erianin phosphate prepared in the present invention;

FIG. 4 is the mass spectrographic analysis spectra of Erianin phosphate prepared in the present invention.

DESCRIPTION OF THE PREFERRED EMBODIMENTS

The Erianin used in this invention is provided by Zhejiang Cell Biomedical Research CO., LTD., whose preparing process can be one of the two below. Other test materials in the present invention are all purchased commercially, unless indicated otherwise. In the following, the two processes of the preparation of Erianin are explained in detail:

1. The process of extracting Erianin from dendrobium

In accordance with the Chinese patent application CN03115752.1, extracting the antineoplastic component Erianin from orchidaceae plant dendrobium by means of supercritical $CO_2$ extraction and column chromatography, with anhydrous alcohol, methanol, and acetone as entrainer and $CO_2$ as extracting medium; then carrying out gel silica column chromatographic separation on the crude product obtained with mixed solution of petroleum benzene and ethyl acetate as eluting reagent and recrystallizing to get the refined extractive which is up to the standard for injection in human body.

2. The process of chemical synthesis of Erianin

In accordance with the process described in the Chinese patent application CN200510083055.4, with 3,4,5-trimethoxybenzaldehyde (i) and isovanillin (iv) as the starting material, the synthetic route for preparing Erianin is as follows:

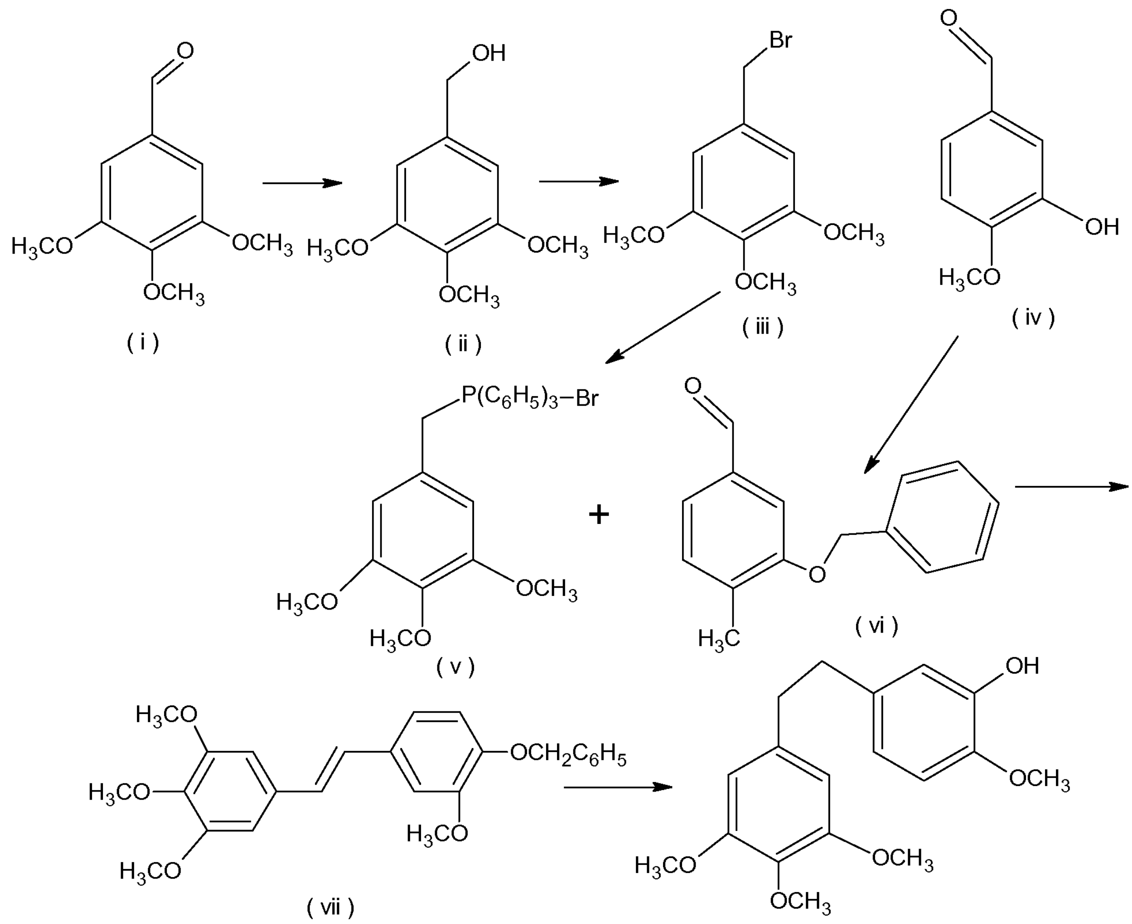
( i ) ( ii ) ( iii ) ( iv ) ( v ) ( vi ) ( vii )

Specifically, the process includes the following steps:

Step 1. Preparation of 3,4,5-trimethoxyl benzyl alcohol (ii)

3,4,5-trimethoxybenzaldehyde (15 g, 76.45 mmol) and anhydrous alcohol (200 ml) were placed in a 250 ml

three-necked flask, and were dissolved at 40°C. Sodium borohydride (1.48 g, 38.23 mmol) was added to the solution. The resulting mixture was heated to reflux for 45 minutes, and monitored by TLC. When the reaction is completed, cooling it to room temperature, deionized water (10 ml, 555.8 mol) was added to quench the reaction. After suction filtering, the filter residue was washed by anhydrous alcohol (20 ml), the combined filtrate was concentrated in rotatory evaporater to dry, dichlormethane (100 ml) was added to dissolve the crude product. The organic layer was washed with sodium hydroxide solution (50 ml) twice and with deionized water (50 ml) twice, and a proper amount of anhydrous magnesium sulfate was added to dry it overnight. After filtering, washing the filter residue with dichlormethane (20 ml). The combined filtrate was concentrated in a rotatory evaporater to afford 3,4,5-trimethoxyl benzyl alcohol, 14.05 g of colorless oily product, yield : 92.72%.

The product does not need to be further purified for following reaction. If pure product is wanted, it can be vacuum distilled for the fraction of distillate of BP 216-218°C/12 mm Hg.

Step 2. Preparation of 3,4,5-trimethoxyl benzyl bromide (iii)

Dissolving 3,4,5-trimethoxyl benzyl alcohol (ii) (14.05 g, 70.89 mmol) in dichloromethane (100 ml) in a 250 ml three-necked flask; phosphorus tribromide (6.73 ml, 70.89 mmol) in dichloromethane (25 ml) was added dropwise and allowed to react at room temperature for 50 minutes, cooling in ice-bath, slowly adding deionized water (18 ml,1.0 mol) dropwise to quench the reaction. The organic layer was washed with deionized water (100 ml) twice, drying with anhydrous magnesium sulfate, filtering, washing the filter residue with dichloromethane (20 ml), the combined organic layer concentrated in rotatory evaporater to dry, and was further dried under vacuum to afford 3,4,5-trimethoxyl benzyl bromide (16.05 g of faint yellow solid), yield: 84.44%.

The product does not need to be further purified for following reaction. If pure product is wanted, it can be recrystallized to get the white lamellar crystal with a 1:3 mixture of ethyl acetate and n-hexane.

Step 3. Preparation of 3,4,5-trimethoxyl benzyl triphenylphosphine bromide (v)

Dissolving 3,4,5-trimethoxyl benzyl bromide (iii) (16.05 g, 61.47 mmol) in toluene (150 ml) in a 250 ml three-necked flask, adding triphenylphosphine (16.12 g, 61.47 mmol) and dissolving immediately. The reaction mixture was heated to reflux for 1 hour, white solid was separated, then cooling to room temperature, suction filtering, the filter cake was washed with toluene (30 ml). After vacuum drying, 3,4,5-trimethoxyl benzyl triphenylphosphine bromide (27.81 g of white powder solid) was isolated, yield: 86.44%.

The product does not need to be further purified for following reaction. If pure product is needed, it can be washed with acetone to get white powder solid.

Step 4. Preparation of isovanillin protected by benzyl group (vi)

Adding isovanillin (vi) (15 g, 98.59 mmol) to anhydrous alcohol (200 ml) in a 250 ml three-necked flask, heating to dissolve at 40°C, adding potassium carbonate (9 g, 65.07 mmol), adding benzylchloride (15 ml, 130.13 mmol) under stirring. The resulting mixture was heated to reflux for 1 hour. After the completion of the reaction (monitored by TLC), cooling it down to 50°C, filtering while hot, cooling the filtrate in refrigerator overnight, the crystal was precipitated, suction filtering, and washing the filter cake with toluene (30 ml). After vacuum drying, the benzyl group protecting isovanillin (white acicular crystal, 19.72 g) was isolated, yield: 82.56%.

The product does not need to be further purified for following reaction. If pure product is needed, it can be recrystallized by absolute alcohol to get white styloid solid.

Step 5. Preparation of Cis/Trans isomer (vii)

Adding 3,4,5-trimethoxyl benzyl triphenylphosphine bromide (v) (20.00 g, 38.21 mmol) and tetrahydrofuran (150 ml) in a 250 ml three-necked flask, stirring the suspension, dissolving isovanillin protected by benzyl group (10.00 g, 41.27 mmol) in tetrahydrofuran (70 ml), and adding it to a dropping funnel (100 ml); adding solid potassium t-butoxide (7.46 g, 66.49 mmol) to the reaction flask, when the reaction system turning to sanguine, stirring for 5 minutes at room temperature, slowly adding the solution of isovanillin protected by benzyl group dropwise, and stirring for 20 minutes at room temperature again. After the completion of the reaction (monitored by TLC), the reaction mixture was transfered into a 500 ml separating funnel, adding deionized water (140 ml), the solution being stratified, extracting with diethyl ether (300 ml) twice, collecting the ether layer, drying with anhydrous magnesium sulfate, filtering, and the filter cake was washed with dry ether (50 ml); concentrating the filtrate in rotatory evaporater to dry to get oily product (25 g); adding absolute alcohol (20 ml) to solidify it, a faint yellow solid (12.50 g) was obtained by suction filtering, yield: 80.48%.

Step 6. Recrystallisation of the cis/trans isomer (vii)

Adding cis/trans isomer (vii) (12.50 g, 30.75 mmol) and anhydrous alcohol (20 ml) in a 50 ml round bottom flask, heating till some solid is dissolved, stirring at room temperature, suction filtering, the filter cake was washed with dry ether (10 ml), and drying by Infrared lamp to afford pure cis/trans product (9.27 g) as faint yellow powder, yield: 74.16%.

Step 7. Preparation of Erianin

Dissolving pure cis/trans isomer (5.14 g, 12.56 mmol) in the mixture of ethyl acetate (100 ml) and absolute alcohol (60 ml) in a 250 ml three-necked flask, the solution being faint yellow, adding 5% Pd-C (0.5 g), stirring while passing hydrogen into the mixture, stirring for 1 hour at room temperature. After filtering, the colorless filtrate was concentrated in a rotatory evaporater to obtain oily product (4.05 g), the crude product of Erianin, yield: 100%.

Step 8. Purification of Erianin

Dissolving 4.05 g (12.72 mmol) of the crude product of Erianin in anhydrous ether (20 ml) in a 50 ml round bottom flask, filtering the insoluble substance (if any), and leaving it in stillness for white crystal to be separated at room temperature, then standing overnight. When the solvent is completely volatilized, a great quantity of white crystal is separated. After suction filtering the filter cake was washed with alcohol to afford white crystal (3.56 g), yield: 100%.

**THE PREPARATION OF ERIANIN SALT**

EXAMPLE 1 The preparation of disodium phosphate of Erianin (converted to sodium salt directly)

Step 1 phosphorylation reaction

Adding phosphorus oxychloride (4.4 ml, 47.4 mmol) and dichloromethane (25 ml) in a 100 ml round bottom flask; adding Erianin (5 g, 15.7 mmol) dropwise to dichloromethane solution (10 ml) and stirring for 5 minutes; adding triethylamine (3.3 ml, 23.8 mmol) dropwise to trichloromethane solution (5 ml). The resulting mixture was stirred for 3 hours at room temperature, monitored by TLC, and the reaction was quenched with cold water (100 ml) after the completion of the reaction; vibrating thoroughly to diverge the organic phase; washing with water (50 ml*2), extracting the water layer with dichloromethane. The combined organic phase was dried with proper amount of anhydrous sodium sulfate overnight; after suction filtering, and the solvent of the filtrate was evaporated under reduced pressure to get a viscous liquid, the crude acyl chloride.

Step 2 Salt forming reaction

Cooling the crude acyl chloride in ice bath, adding sodium hydroxide solution (2 mol/L) while stirring till the pH value of the mixture is between 8 ~ 10, stirring for 8 hours at 50°C ~ 80°C, filtering to remove the insoluble substance, decompressing to evaporate most of the solvent, cooling for the white solid to be separated, which is the crude disodium phosphate of Erianin. Dissolving the crude product with ethanol while heated, filtering to remove the insoluble solid while hot, and cooling the filtrate for separated crystal as pure disodium phosphate of Erianin (6.0 g), a white crystal product with a yield of 86% m.p. 266-269°C, (splitting), dissolubility in water: 200 mg/ml.

Step 3 Physical property test: drawings 1~4 are the analytic results of nuclear magnetic resonance, infrared and mass spectra of the product prepared.

$^1$HNMR ($D_2O$): δ2.77(d,1H,J=13.7Hz,H-1α'), 2.81(d,1H,J=13.7 Hz,H-1α), 3.67(s,3H,4'=$OCH_3$), 3.69 (s,6H,3,5-$OCH_3$), 3.75(s,3H,4-$OCH_3$), 6.47(s,2H,H-2,6), 6.77(d,1H,J=8.36Hz,H-5'), 6.79(d,1H,J=8.36Hz,H-6'), 7.27(d,1H,J=1.8 Hz, H-2').
$^{13}$CNMR($D_2O$) : δ152.27, 148.05, 148.00, 143.35, 139.54, 135.14, 134.71, 122.32, 120.63, 120.62, 112.89, 106.21, 61.01, 56.30, 56.09, 37.590, 36.70;
IR(KBr) : υ3852w, 3385s, 2938m, 2839m, 1588m, 1509m, 1464m, 1420m, 1273m, 1124s, 987m, $cm^{-1}$.
ESIMS : m/z:396.9(M+). Calc. 441.97 for $C_{18}H_{22}O_8PNa_2$.

EXAMPLE 2 The preparation of disodium phosphate of Erianin (with the formation of the intermediate of organic phosphate)

Step 1 Synthesis of Erianin phospho-dibenzyl ester

Dissolving Erianin (20 g, 63.2 mmol) in acetonitrile (200 ml) with argon shield in a dry three-necked flask, stirring and cooling to -25°C, adding $CCl_4$ (35 ml, 316 mmol), stirring for 5 minutes, adding diisopropylethylamine (23.13 ml, 133 mmol) dropwise by syringe, then adding DMAP (772 mg, 6.32 mmol), adding dibenzyl phosphorite (20.33 ml, 92 mmol) dropwise 1 minute later, keeping the reacting temperature below -10°C, monitored by TLC after 1 hour, if reaction was completed, adding 50 ml $KH_2PO_4$ (0.5 M), extracting with ethyl acetate (100 ml $\times$ 3), the combined organic layer was washed with water (100 ml) and saturated NaCl (100 ml). After drying, filtering, and evaporating the solvent to get a yellow oily product; which was purified column chromatography (ethyl acetate: hexane=2: 3) and recrystallizing by ethyl acetate-hexane to get the colorless acicular product, Erianin phospho-dibenzyl ester (m.p 73°C).

Step 2 Preparing sodium phosphate of Erianin

Stirring the acetonitrile solution (100 ml) of Erianin phospho-dibenzyl ester (20.5 g, 35.6 mmol) and NaI (10.7 g, 71 mmol) vigorously under the protection of argon, adding trimethyl chlorosilane silicane (9.02 ml, 71 mmol) slowly, stirring for 20 minutes, no starting material point particle was detected by TLC, adding water (just enough to dissolve the salt), adding sodium thiosulfate (10%, 2 ml), separating the organic phase, extracting the water layer by ethyl acetate (50 ml $\times$ 4), the combined organic layer, was concentrated under vacuum to get a yellow spumescent product.

Dissolving the spumescent product in methanol (100 ml), adding sodium methylate (95%, 4.1 g, 71 mmol), stirring for 9 hours, the methanol was evaporated under vacuum, and recrystallizing the solid with water-acetone and methanol-acetone to get a white solid (m.p. 266-269°C), disodium phosphate of Erianin.

The results of physical property determination to disodium phosphate Erianin are the same as those of example 1. It is clear that the final product prepared by the process in current invention is disodium phosphate of Erianin.

EXAMPLE 3 The preparation of Erianin sulphate salt

Adding Erianin (10 mmol) and N, N-dimethylaniline (75 mmol) in a 250 ml round bottom flask, adding $CH_2Cl_2$ equal volume to N,N-dimethylaniline, stirring in ice-salt bath (-5~-10°C), and adding dropwise chlorosulfonic acid (12.5 mmol); then continuing to stir for 1 hour in brine bath, removing the brine bath, stirring for 24 hours at room temperature, adjusting the PH value of reaction liquid to 10 with NaOH (10 mol/l) under stirring. Then the resulting mixture was cooled in refrigerator. After filtration, the filtrating residue was washed with dry ether, dissolving the solid product in methanol and it was purified by column chromatography after drying, and evaporating the solvent to get a white solid (hydroscopic), sodium sulfate of Erianin.

Physical property test:

$^1H$ NMR ($D_2O$): $\delta$2.79(d,1H,J=13.7Hz,H-1$\alpha$'), 2.80(d,1H,J=13.7 Hz,H-1$\alpha$), 3.67(s,3H,4'=$OCH_3$), 3.74 (s,6H, 3,5-$OCH_3$), 3.75(s,3H,4-$OCH_3$), 6.55 (s,2H,H-2,6), 6.58(d,1H,J=8.36Hz,H-5'), 6.85(d,1H,J=8.36Hz,H-6'), 7.33(d,1H, J=1.8 Hz, H-2'). $^{13}$CNMR($D_2O$): $\delta$152.27, 148.05, 148.00, 143.35, 139.54, 135.14, 134.71, 122.32, 120.63, 114.33, 108.03, 106.21, 61.01, 56.30, 56.09, 37.590, 36.70;

IR(KBr) : $\upsilon$3539s, 3433m, 3001w, 2939m, 2841m, 1589s, 1508s, 1466m, 1422m, 1330m, 1262m, 1242s, 1174m, 1131s, 1014m, 619m, $cm^{-1}$.

MS(ESI) : m/z: 398.427 (M+). Calc. 421.46 for $C_{18}H_{22}O_8SNa$.

**SAFETY EVALUATION OF ERIANIN SALT**

EXAMPLE 4 The LD50 determination of disodium phosphate of Erianin

1. Material

Experimental animal: Kunming mice, provided by Shanghai Slac Laboratory Animal Co., Ltd., weight being between 18~22g, half and half of female and male.

Reagent: 0.9%NaCl , disodium phosphate of Erianin (AP), provided by Zhejiang Cell Biomedical Research Co., Ltd., which is prepared by the process above, bitter acid.

Equipment: one-off syringe (1ml)

2. Experimental method and procedure

(1) Experimental animal: Kunming (KM) mice, set up in 7 groups, 10 mice every group and half and half of female and male. Marked by bitter acid.
(2) In every group, administering by vena caudalis injection according to body weight of 1300 mg/kg, 1250 mg/kg, 1200 mg/kg, 1150 mg/kg, 1100mg/kg, 1050mg/kg, 1000mg/kg.
(3) Observing at 0.25h, 0.5h, 1h, 2h, 4h, 24h respectively after administration and recording the death rate; then observing once every day and recording the death rate (table 1) for 14 days; putting the mice still alive to death on the 15th day and giving pathological anatomy.

Table 1 dosage-death rate

| dosage (mg/kg ) | Deaths | | | | | | | death rate in total |
|---|---|---|---|---|---|---|---|---|
| | hours | days | | | | | | |
| | 0-4 | 1 | 2 | 3 | 4 | 5 | 6 | |
| 1300 | | 6 | | 1 | | | | 7 |
| 1250 | 1 | 3 | 2 | | | | | 6 |
| 1200 | 1 | | | | | 1 | 1 | 3 |
| 1150 | | 1 | | | | | | 1 |
| 1100 | | 3 | | | | | | 3 |
| 1050 | | 1 | | | | | | 1 |
| 1000 | | | | | | | | 0 |

3. Experimental result

LD50 ofAP = 1243.0377 mg/kg Confidence interval : 1049.9757≤LD50≤1471.5984

Anatomy showed no obvious pathological change in the mice died in the process of the experiment, and pathological anatomy showed no pathological change in the mice executed after 14 days either.

It means that the toxicity of 3,4,5,4'-tetramethoxyl diphenylethane-3'-O-disodic phosphate (AP) is very low, and is very safe in clinical medication.

**THE PHARMACODYNAMICS EXPERIMENT FOR ERIANIN SALT AS ANTINEOPLASTIC**

EXAMPLE 5 The antineoplastic application of disodium phosphate of Erianin

1. Experimental samples

Samples: disodium phosphate of Erianin (AP, prepared by the process of example 1) prepared the concentration needed with physiologic saline.

Control medicine: cyclophosphamide for injection (CTX), Shanghai Hualian Pharmaceutical Co., Ltd, batch number: 020806. dissolved with physiologic saline for use.

2. Animals and tumor strains

Fifty Kunming mice, female, weight: 19 ± 1 g, provided by the animal house in Shanghai Institute of Pharmaceutical Industry.

Two S180 ascites tumor mice, provided by the animal house in Shanghai Institute of Pharmaceutical Industry.

3. Testing method

Drawing ascites under aseptic condition from S 180 ascites tumor mice, counting the cells, diluting to 1-2x107/ml with physiologic saline, giving subcutaneous inoculation in the armpit of the mice with 0.2 ml each, randomly dividing the mice equally into five groups the next day, ten in each group.

Dosage for administration (mg/kg./d): AP 25 mg/kg, 75 mg/kg, 225 mg/kg
Means of administration: intravenous injection
The control medicine, cyclophosphamide, administered by intravenous injection, the dosage being 30 mg/kg×9d.
And setting up blank control group.
Administering medicine to the mice by weight the next day of inoculation, intragastric administration 0.5 ml/20 g for 9 days, by weight, and putting them to death on the tenth day after inoculation, weighing the tumor and calculating the rate of tumor inhibition.
Assessing the result according to the following formula:

$$\text{the rate of tumor inhibition} = \frac{\text{Tumor weight of control group} - \text{Tumor weight of administration group}}{\text{Tumor weight of control group}} \times 100\%$$

4. Result

No death in all groups during the experimental session

Table 2. The experimental result of the inhibiting effect of AP to S 180 tumor-bearing mice

| samples | dosage mg/kg | Means of Administration | Animal counts | Weight 0d (g) | Weight 10d (g) | Tumor weight | rate of tumor inhibition % |
|---|---|---|---|---|---|---|---|
| AP | 25 | i.v | 10/10 | 18.37±1.58 | 22.93±1.28 | 1.42±0.26 | 42 |
| AP | 75 | i.v | 10/10 | 18.41±1.74 | 22.75±2.25 | 0.82±0.20 | 67 |
| AP | 225 | i.v | 10/10 | 19.65±1.26 | 22.01±3.46 | 0.69±0.08 | 72 |
| CTX | 30 | i.v | 10/10 | 19.78±1.20 | 19.58±2.54 | 0.41±0.17 | 83 |
| Control | | | 10/10 | 20.29±1.60 | 23.34±1.87 | 2.45±0.27 | |

INDUSTRIAL APPLICATION

The Erianin salt provided in the present invention has better water-solubility then Erianin, so its preparation is easier, the in vivo bioavailability of the medication is higher, and it has an antineoplastic effect of high efficacy and low toxicity.
In the preparing process of the present invention, all agents are ordinary products of chemical industry that are cheap and easy to obtain, and the route of synthesis is short and the operation is easy, suitable for industrial mass production.
The above description of the preferred embodiments of this invention does not limit this invention, and those skilled in the art may make various changes and modifications in accordance with this invention, which, as long as they do not break away from the spirit of this invention, shall be within the scope of the claims of this invention.

**Claims**

**1.** A pharmaceutically acceptable salt of Erianin represented by the following general formula (I):

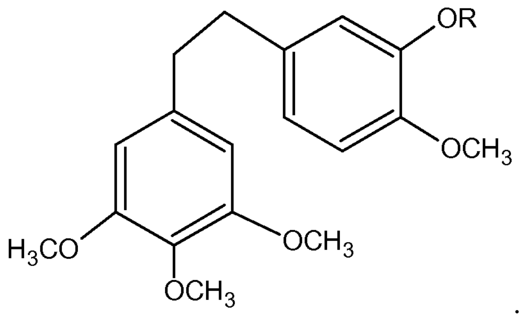

(I)

wherein:

R is a salt formed by monobasic acid radical of inorganic oxacid combining with metals, ammonium salts, or organic amine;
said metals are selected from a group consisting of alkali metals and alkali-earth metals;
said inorganic oxacid is selected from a group consisting of phosphoric acid, sulphuric acid, sulfurous acid, nitric acid, carbonic acid, hypochloric acid and trifluoroacetic acid.

**2.** The pharmaceutically acceptable salt of Erianin according to claim 1, wherein said pharmaceutically acceptable salt of Erianin is an organic phosphate of Erianin or a sulphate of Erianin.

**3.** The pharmaceutically acceptable salt of Erianin according to claim 1 or 2, wherein said pharmaceutically acceptable salt of Erianin is an organic phosphate of Erianin represented by the following general formula (II):

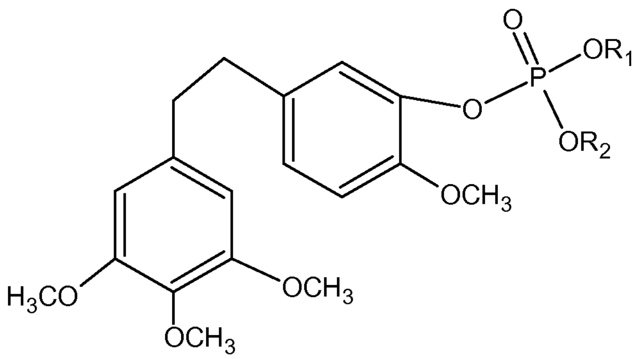

(II)

wherein $R_1$, $R_2$ are independently H, Na, K, or $NH_4$ respectively, and $R_1$ and $R_2$ are not H simultaneously.

**4.** The pharmaceutically acceptable salt of Erianin according to claim 3, wherein in formula (II), $R_1$ is Na, $R_2$ is Na, and the said Erianin salt is disodium organic phosphate of Erianin.

**5.** The pharmaceutically acceptable salt of Erianin according to claim 1 or 2, wherein said pharmaceutically acceptable salt of Erianin is sulphate salt of Erianin represented by the following general formula (III):

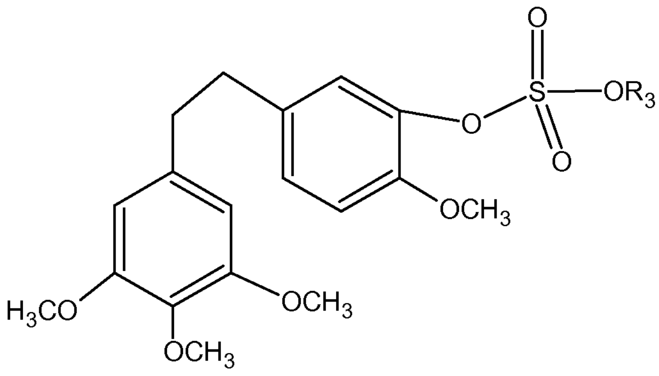

(III)

wherein: $R_3$ is Na, K, or $NH_4$.

**6.** The pharmaceutically acceptable salt of Erianin according to claim 5, wherein in formula (III), $R_3$ is Na, and the said Erianin salt is sodium sulphate of Erianin.

**7.** A process for preparing a pharmaceutically acceptable salt of Erianin comprising the following steps:

A1. reacting Erianin with phosphorylating agent by phosphorylating reaction of the phenolic hydroxyl in the presence of an acid binding agent to form compound (IV), the reacting temperature being 25~80°Cand the reacting solvent being an inert organic solvent;
A2. reacting the reaction liquid obtained in step A1 with alkali directly to obtain compound (II), an organic phosphate salt of Erianin;
the synthetic route being:

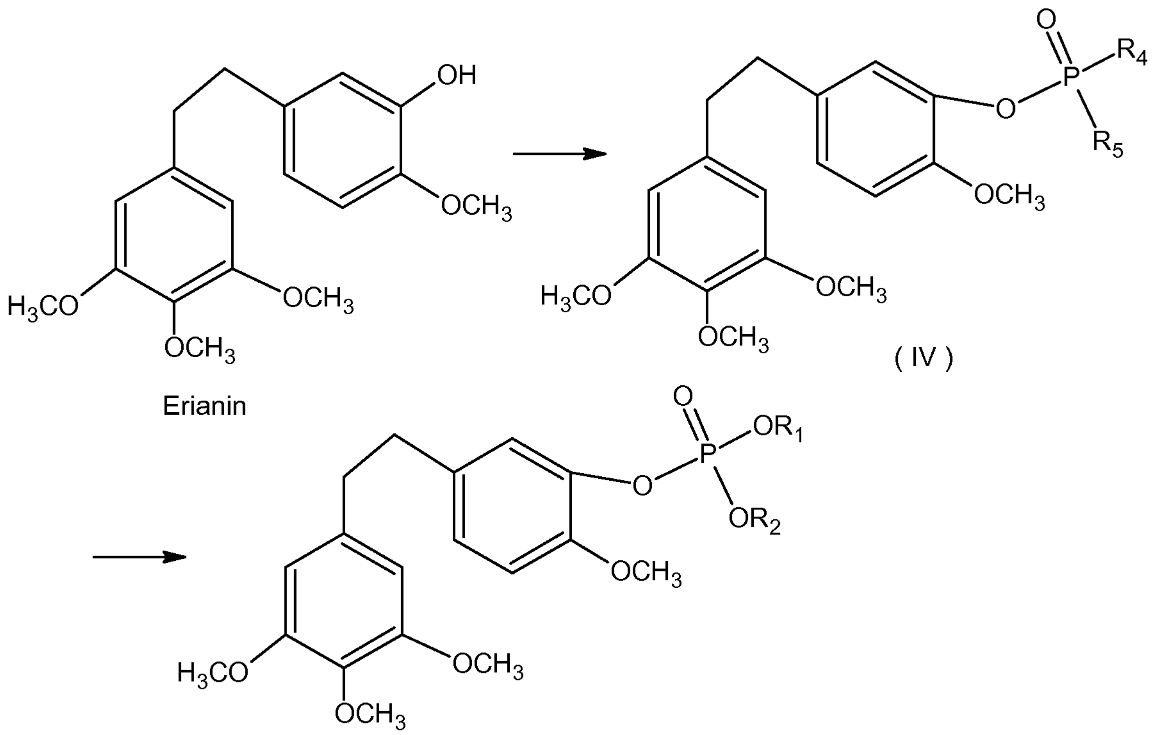

Erianin

(IV)

(II)

wherein

in formula (IV), $R_4$, $R_5$ are independently Obu$^t$, Cl or Br respectively, wherein bu$^t$ represents tert-butyl group; in formula (II), R1, R2 are independently H, Na, K, or $NH_4$ respectively, and $R_1$ and $R_2$ are not H simultaneously

**8.** The process according to claim 7, wherein in step A1:

said phosphorylating agent is selected from a group consisting of $OPCl_3$, $OPBr_3$, and $(Bu^tO)_2P(O)Cl$;
said acid binding agent is selected from a group consisting of pyridinium, monalkylamine, dialkylamine and tri alkylamine;
said inert organic solvent is selected from a group consisting of dichloromethane, dichloroethane, benzene, carbon tetrachloride and acetonitrile.

in step A2:

said alkali is selected from a group consisting of NaOH, KOH, $NaOCH_3$, $KOCH_3$, $NaOCH_2CH_3$, and $KOCH_2CH_3$.

**9.** The process according to claim 7 or 8, wherein in step A1, the phosphorylating agent is $OPCl_3$, and the acid binding agent is triethylamine; in step A2, the alkali is NaOH.

**10.** A process for preparing a pharmaceutically acceptable salt of Erianin, comprising the following steps:

B1. under the protection of inert gas, reacting Erianin with dibenzylphosphorite in the presence of acid binding agent by phosphorylating reaction of the phenolic hydroxyl to obtain compound (V), the reaction temperature being -40°C~-10°C, the reaction being carried out in inert organic solvent;
B2. under the protection of inert gas, reacting compound (V) with NaI in the presence of active group protection agent to obtain compound (VI), the reaction being carried out in inert organic solvent;
B3. reacting compound (VI) with alkali to obtain compound (II),
the synthetic route is:

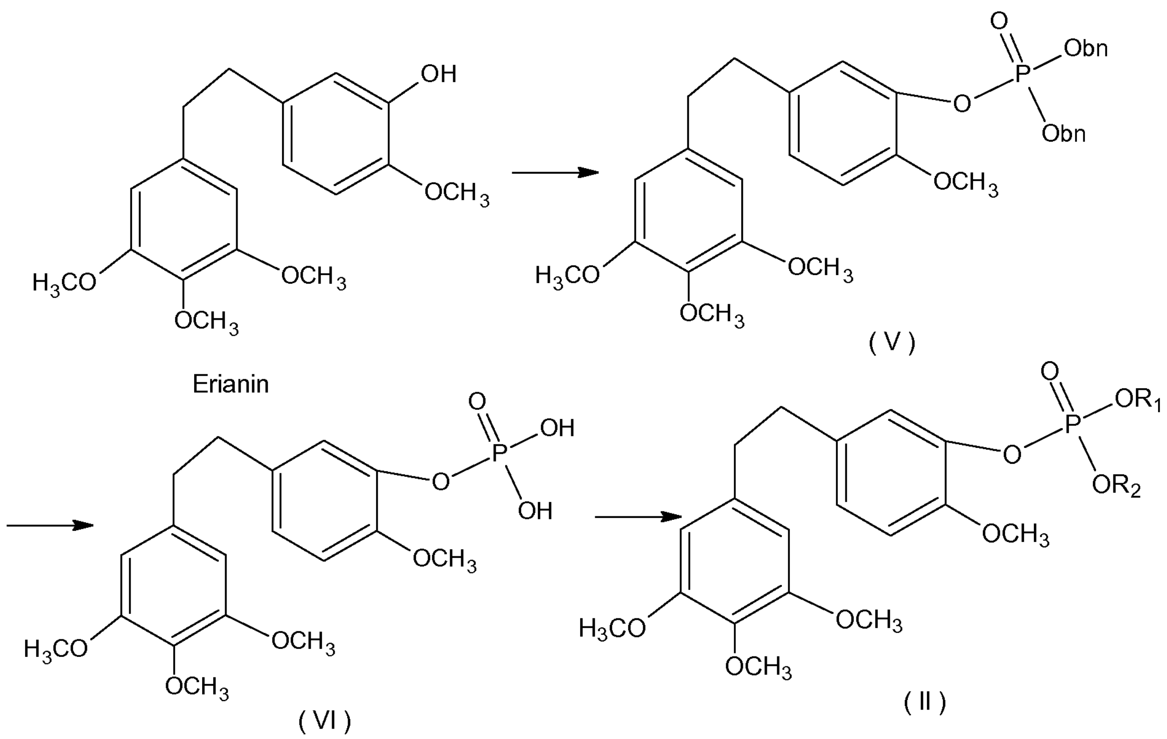

Erianin

( V )

( VI )

( II )

wherein in formula (V), bn represents benzyl group; in formula (II), $R_1$, $R_2$ are independently H, Na, K, or $NH_4$ respectively, and $R_1$ and $R_2$ are not H simultaneously.

**11.** The process according to claim 10, wherein in step B1 :

said acid binding agent is selected from a group consisting of pyridinium, monalkylamine, dialkylamine, trialkylamine and dimethylamino pyridine;
said inert gas is argon, nitrogen gas or carbon monoxide;
said inert organic solvent is selected from a group consisting of dichloromethane, dichloroethane, benzene, carbon tetrachloride and acetonitrile;

in step B2:

said inert organic solvent is selected from a group consisting of dichloromethane, dichloroethane, benzene, carbon tetrachloride and acetonitrile;

in step B3:

said alkali is selected from a group consisting of NaOH, KOH, $NaOCH_3$, $KOCH_3$, $NaOCH_2CH_3$ and $KOCH_2CH_3$.

**12.** The process according to claim 10 or 11, wherein in step B1,
said acid binding agent is diisopropylethylamine and dimethylamino pyridine, and the inert gas is argon;
in step B2, the inert gas is argon, the protecting agent of active group is trimethyl chloride silane, and the inert organic solvent is acetonitrile and/or carbon tetrachloride;
in step B3, the alkali is sodium hydroxide .

**13.** A process for preparing a pharmaceutically acceptable salt of Erianin, comprising the following steps :

C1. reacting Erianin with halo sulfonic acid by sulfonylating reaction of phenolic hydroxyl in the presence of an acid binding agent to obtain compound (VII), the reacting temperature being -5~-10°C and the reacting solvent being an inert organic solvent;
C2. reacting the reacting liquid obtained in step C1 with alkali directly to prepare compound (III);
the synthetic route is:

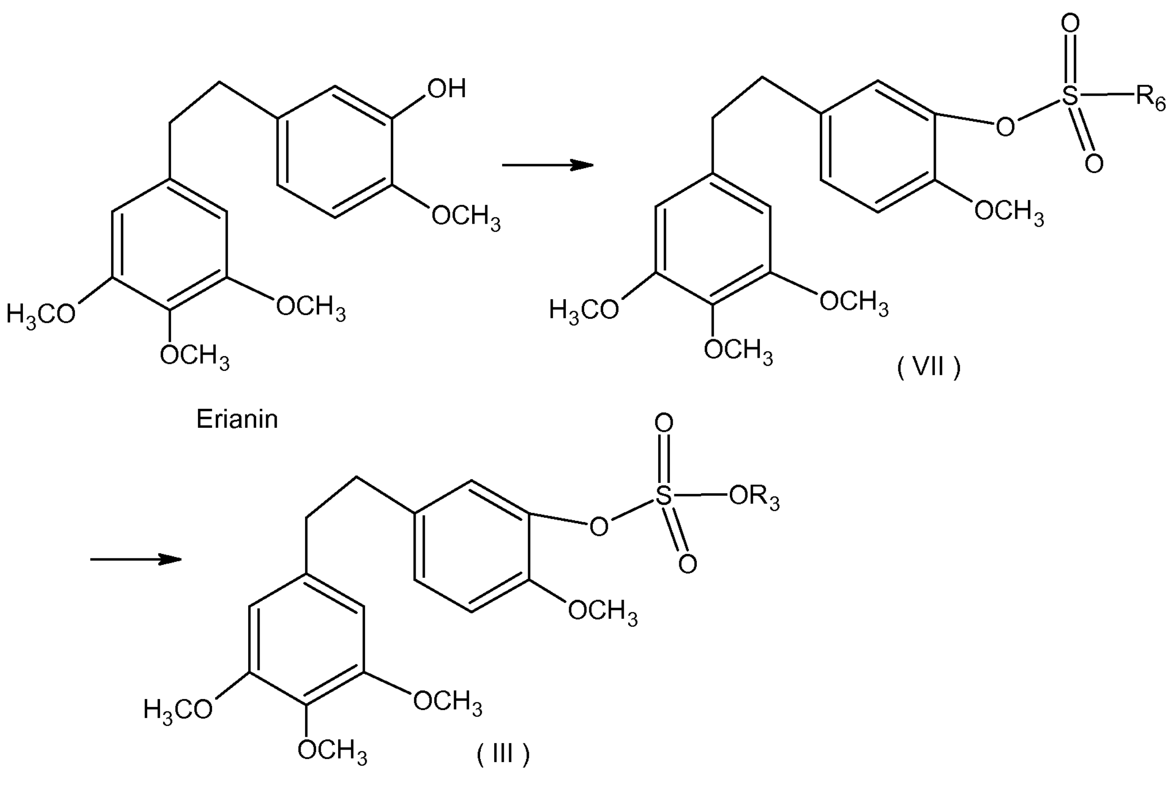

Erianin

( VII )

( III )

wherein in formula (VII), $R_6$ is Cl or Br; in formula (III), $R_3$ is Na, K, or $NH_4$.

**14.** The process according to claim 13, wherein in step C1:

said halogen sulfonic acid is chlorosulfonic acid or bromo sulfonic acid;
said acid binding agent is selected from a group consisting of pyridinium, monalkylamine, dialkylamine and trialkylamine;
said inert organic solvent is selected from a group consisting of dichloromethane, dichloroethane, benzene, and carbon tetrachloride;

in step C2:

said alkali is selected from a group consisting of NaOH, KOH, $NaOCH_3$, $KOCH_3$, $NaOCH_2CH_3$ and $KOCH_2CH_3$.

**15.** The process according to claim13 or 14, wherein in step C1,
the halo sulfonic acid is chlorosulfonic acid, the acid binding agent is N, N-dimethylaniline, and the inert organic solvent is dichloromethane;
in step C2, the alkali is NaOH.

**16.** A pharmaceutical composition comprising an efficient amount of a pharmaceutically acceptable salt of Erianin according to anyone of claims 1 to 6 in amount effective to treat diseases together with a pharmaceutically acceptable carrier and/or excipient.

**17.** The pharmaceutical composition according to claim16, wherein said pharmaceutical composition is in the form of preparation for injection or oral administration.

**18.** A use of the pharmaceutically acceptable salt of Erianin according to anyone of claims 1 to 6 in preparing an antitumor pharmaceutical.

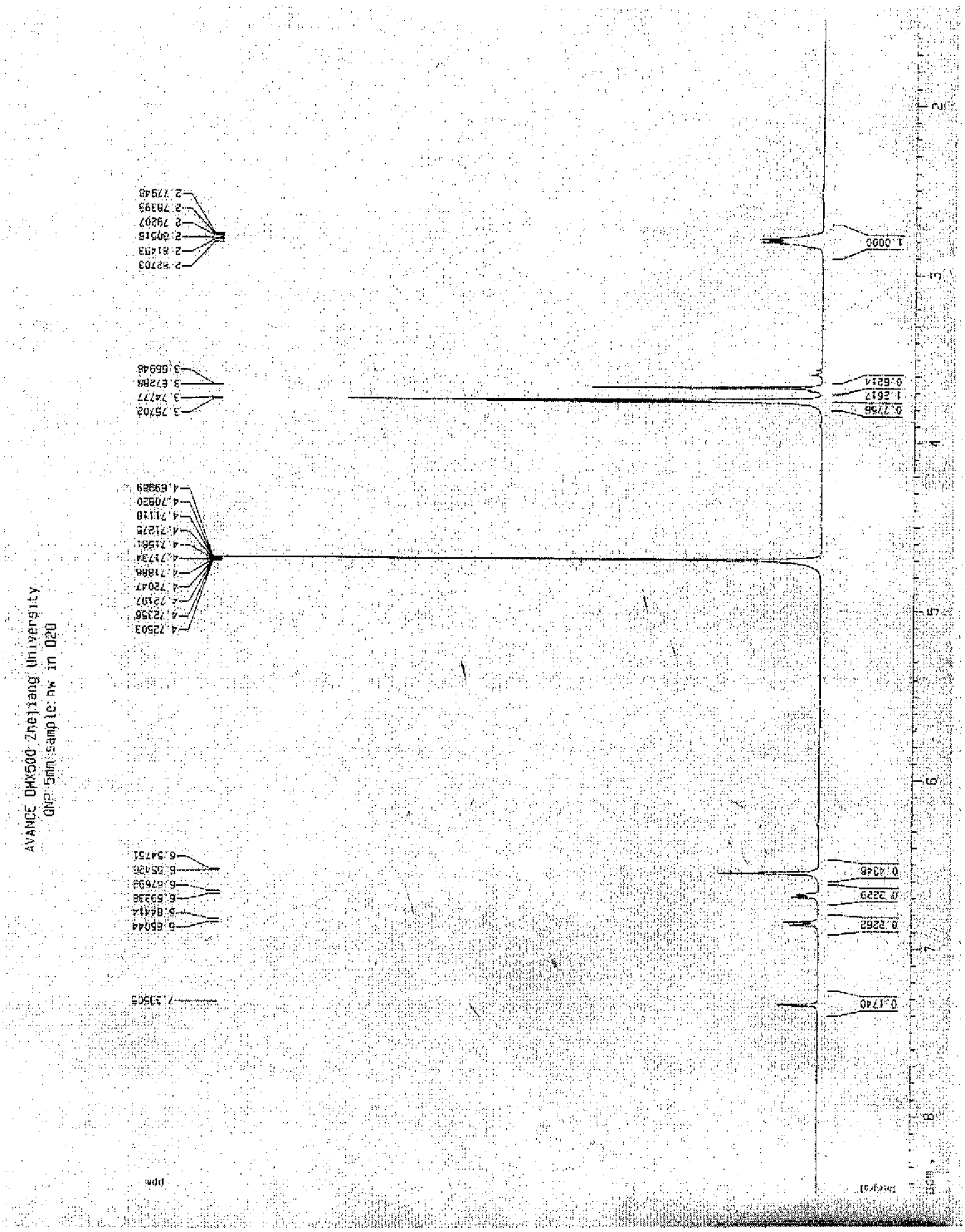

FIG 1

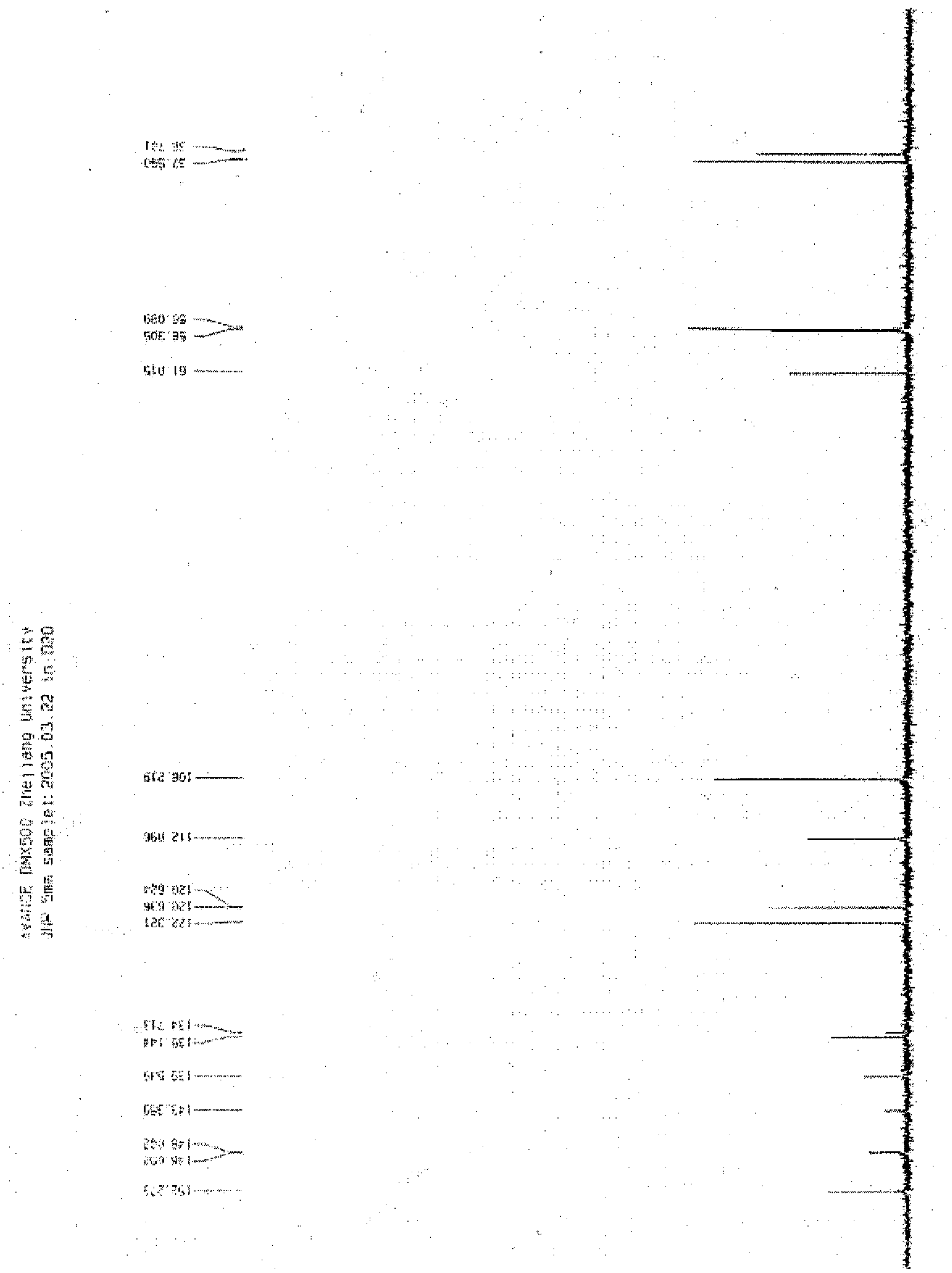

FIG 2

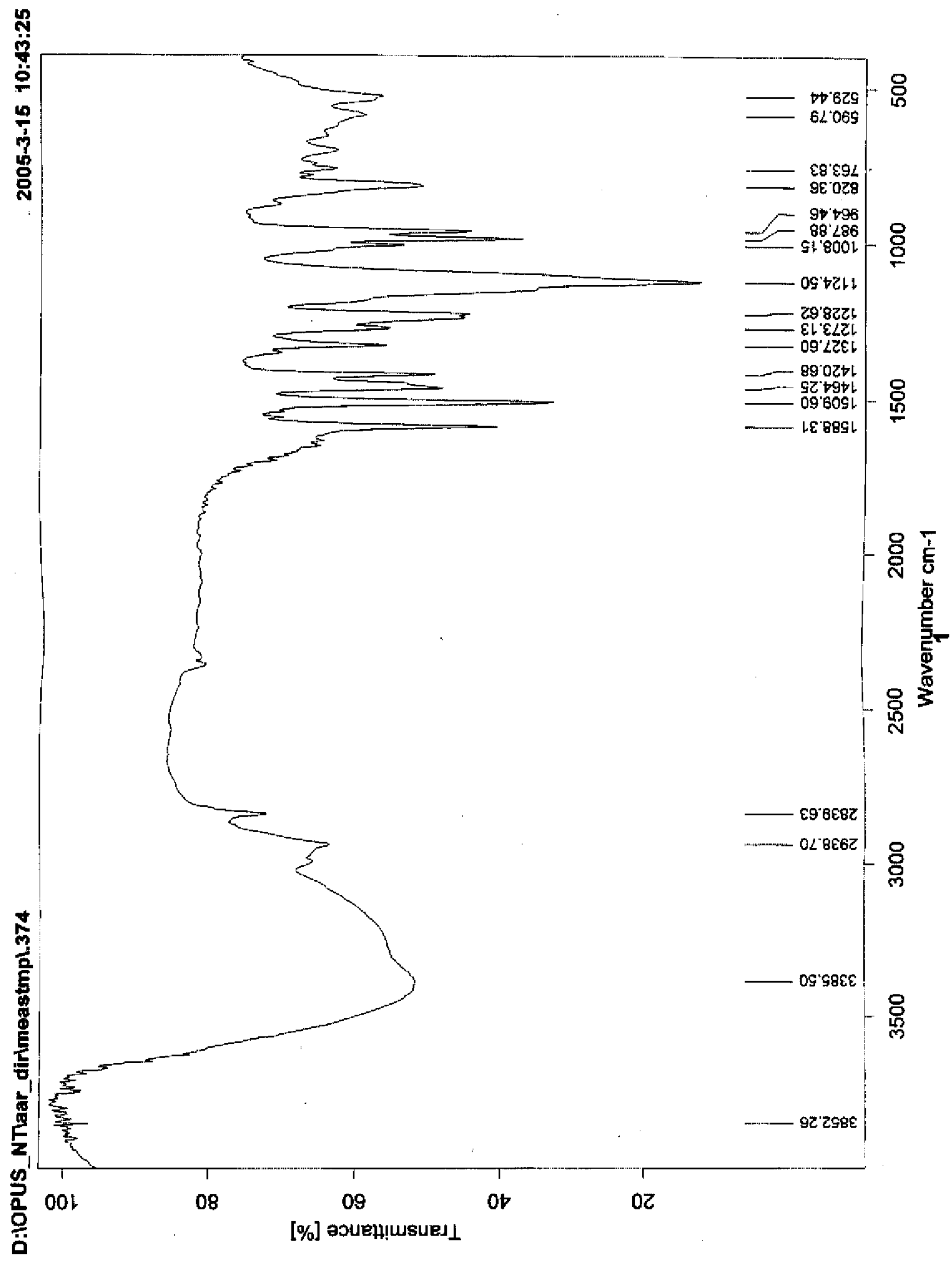
D:\OPUS_NT\aar_dir\meastmp\.374
2005-3-15 10:43:25
Transmittance [%]
Wavenumber cm-1
3852.26
3385.50
2938.70
2839.63
1588.31
1509.60
1464.25
1420.68
1327.60
1273.13
1228.62
1124.50
1008.15
987.88
964.46
820.36
763.83
590.79
529.44

FIG 3

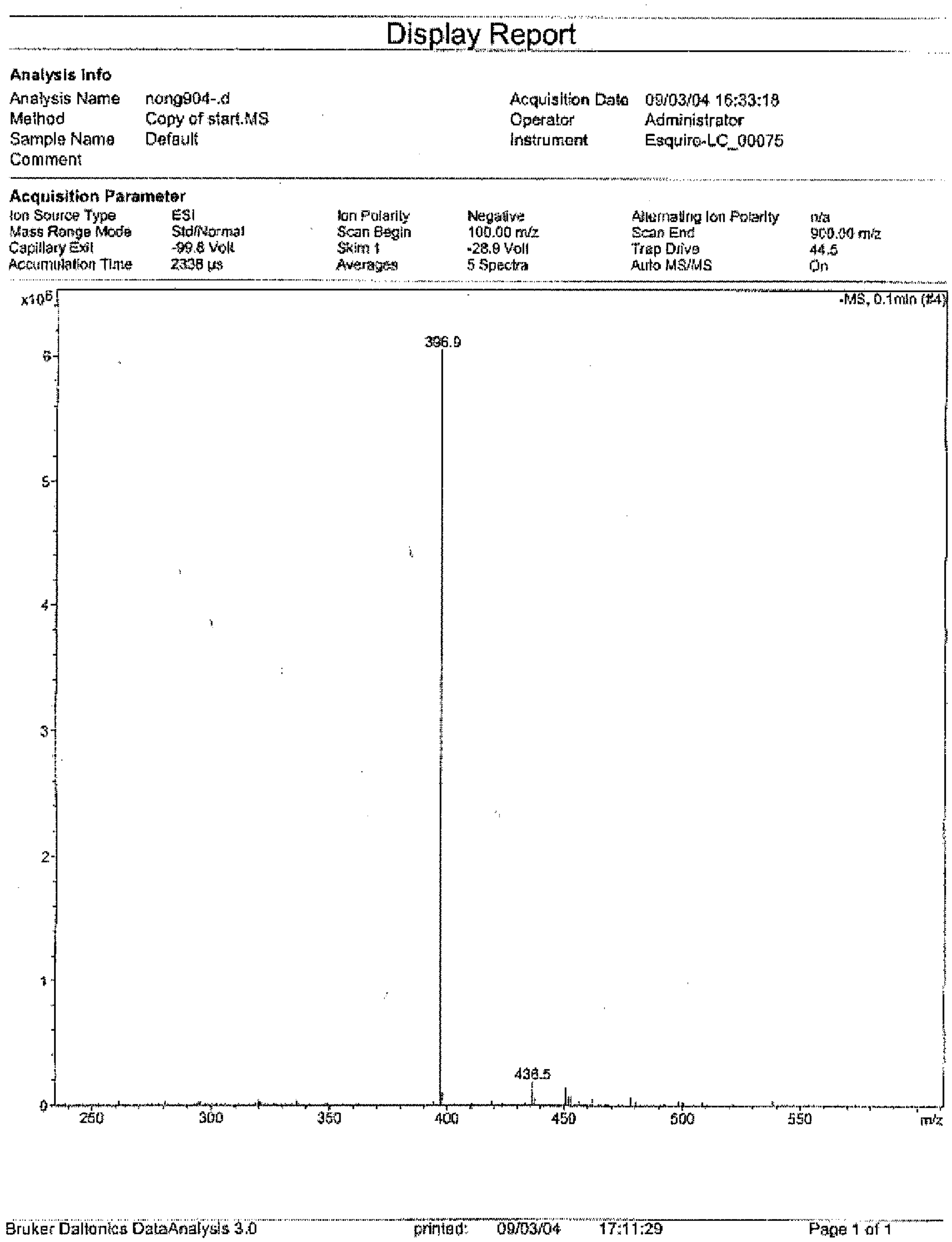
Display Report
Analysis Info
Analysis Name nong904-.d
Method Copy of start.MS
Sample Name Default
Comment
Acquisition Date 09/03/04 16:33:18
Operator Administrator
Instrument Esquire-LC_00075
Acquisition Parameter
Ion Source Type ESI
Mass Range Mode Std/Normal
Capillary Exit -99.8 Volt
Accumulation Time 2338 µs
Ion Polarity Negative
Scan Begin 100.00 m/z
Skim 1 -28.9 Volt
Averages 5 Spectra
Alternating Ion Polarity n/a
Scan End 900.00 m/z
Trap Drive 44.5
Auto MS/MS On
x10^5
-MS, 0.1min (#4)
396.9
436.5
0
1
2
3
4
5
6
250
300
350
400
450
500
550
m/z
Bruker Daltonics DataAnalysis 3.0
printed: 09/03/04 17:11:29
Page 1 of 1

FIG 4

# INTERNATIONAL SEARCH REPORT

International application No.
PCT/CN2006/001918

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

C07F 9 C07C43 A61K31 A61P35

Documentation searched other than minimum documentation to the extent that such documents are included in the fields searched

Electronic data base consulted during the international search (name of data base and, where practicable, search terms used)

CNPAT, EPODOC, WPI, PAJ, CNKI, CAPLUS, REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | Wang, Tianshan et al., "In vitro inhibition of leukemia $K_{562}$ cells growth by constituents from Dendrobium chrysotoxum", Tianran Chanwu Yanjiu Yu Kaifa, 1997, Vol.9, No.2:1-3 | 1-18 |
| A | Nandy, Partha. et al., "Quantitative structure-activity relationship analysis of combretastatins: a class of novel antimitotic agents", Pharmaceutical Research, 1991, Vol.8, No.6:776-81, see the compound 15 | 1-18 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 11. Oct 2006 (11.10.2006) | 16 · NOV 2006 (16 · 11 · 2006) |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>Xu Chi<br>Telephone No. 86-10-62085562 |

Form PCT/ISA /210 (second sheet) (April 2005)

## INTERNATIONAL SEARCH REPORT

International application No.
PCT/CN2006/001918

CLASSIFICATION OF SUBJECT MATTER

C07F 9/08 (2006.01) i
C07C 43/235 (2006.01) i
A61K 31/09 (2006.01) i
A61P 35/00 (2006.01) i

Form PCT/ISA /210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 03115752
- CN 200510083055


### Non-patent literature cited in the description


- **WANG TIANSHAN.** In vitro Inhibition Activities on the Growth of Tumor Cell Strain K256 by Constituents from Dendrobium Chrysotoxum. *Natural Product Research and Development,* 1997, vol. 9 (2), 1-3
- Inhibitory effects of dendrobium chrysotoxum and its constituents on the mouse HePA and ESC. *Journal of China Pharmaceutical University,* 1994, vol. 25 (3), 188-189
- **LI YUNMAN.** Erianin Induces Apoptosis of Human leukemia HL-60 cells. *Acta Pharmacologica Sinica,* November 2001, vol. 22 (11), 1018-1022